# EUROPEAN PATENT SPECIFICATION

(11) **EP 0 478 803 B1**
(45) Date of publication and mention of the grant of the patent: **10.07.1996**
(21) Application number: 91908476.4
(22) Date of filing: 19.04.1991
(51) Int. Cl.: C07D 307/58

(54) **Process for producing (S)-gamma-acyloxy-methyl-alpha-beta-butenolide**
Verfahren zur Herstellung von (S)-Gamma-Acyloxy-Methyl-alpha-beta-Butenolid
Procédé de production de (S)-gamma-acyloxy-methyle-alpha-beta-butenolide

(30) Priority: 19.04.1990 JP 101843/90
(43) Date of publication of application: 08.04.1992
(73) Proprietor: Japan Tobacco Inc., Minato-Ku Tokyo 105 (JP)
(72) Inventor: SHIBAGAKI, M. Jap. Tob. Inc. Life Science Res.Lab, Kanagwa-kenh227 (JP); HONDA, I. Jap. Tob.Inc. Life Science Res.Lab, Kanagwa-ken 227a- (JP); TAKAHASHI, K. Jap. Tob.Inc. Life Science Res. Lab, Kanagwa-ken227 n 227 (JP); KUNO, H. Jap.Tob. Inc. Life Science Res. Lab., Kanagwa-ken 227 a (JP); MORI, M. Jap.Tob.Inc. Life Science Res. Lab., Kanagwa-ken 227 (JP); MATSUSHITA, H. Jap. Tob. Inc. Life ScienceRes., Kanagwa-ken o227 ken 227 (JP)
(74) Representative: Reinhard - Skuhra - Weise & Partner
(86) International application number: JP9100522
(87) International publication number: WO9116319

(56) References cited:
- EP-A- 0 411 403
- JP-A-63 222 162
- CHEMICAL ABSTRACTS, vol. 113, no. 11, 10 September 1990, Columbus, Ohio, US; abstract no. 97347F, K. KOSEKI ET AL.: 'A method for easy preparation of optically pure (S)-5-Hydroxy-2-penten-4-olide and (S)-5-Hydroxypentan-4-olide' page 684 ;column 1 ;

## Description

### [Technical Field]

This invention relates to a method for producing (S)-γ-acyloxymethyl-α,β-butenolide of the following chemical formula:

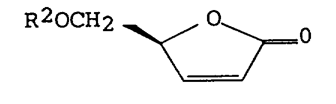

The subject compound described above is useful as a starting material for synthesis of pharmaceutical drugs such as prostaglandin, antileukemic lignan, and antibiotic rasaroside A. The compound is also useful as an intermediate for synthesis of 2,3-dideoxyribose derivatives.

### [Background Art]

In order to synthesize (S)-γ-acyloxymethyl-α,β-butenolide, the following two methods are known. However, each method suffers from the disadvantages as follows.

One is a method to convert D-ribose of a starting material through ribolactone into the subject compound (P.Camps et al., Tetrahedron, 38,2395 (1982)).

This first method utilizes an expensive ribose as a starting material, cost for the product is therefore disadvantageously increased.

Another is a method to utilize L-glutamic acid as a starting material. In this method, L-glutamic acid is oxidized to the subject compound through γ-lactone via six steps (M.TANIGUCHI et al., Tetrahedron, 30,3547 (1974)). This second method disadvantageously requires complicated reaction steps. Moreover, since this method can produce racemic mixture during the reaction, disadvantages such as reduced yield are occurred.

### [Disclosure of Invention]

An object of the invention is to provide a method which overcomes the disadvantages of the conventional methods as stated above and which can easily produce (S)-γ-acyloxymethyl-α,β-butenolide at lower cost.

The inventors of the invention have energetically investigated and found a method which utilizes (6S)-2-aryloxy- and (6S)-2-alkoxy-6-acyloxymethyl 2H-pyran-3-(6H)-one as starting materials.

Specifically, this invention is a method for producing (S)-γ-acyloxymethyl-α,β-butenolide by oxidizing 2-alkoxy-6-acyloxymethyl 2H-pyran-3(6H)-one in an organic solvent with peracid.

Detailed description of the invention is as follows.

According to the invention, a reaction in which a starting material (I) is oxidized with peracid to afford a subject compound (II) is shown by the following formula. This reaction is considered to include Baeyer-Villiger reaction.

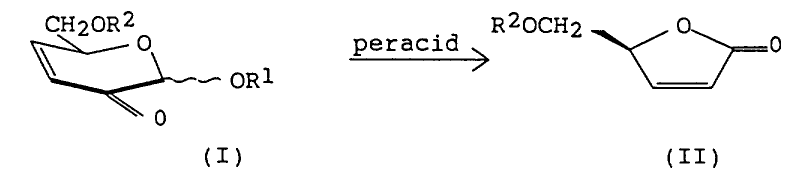

Examples of R¹ of the starting material (I), i.e., (6S)-2-aryloxy- and (6S)-2-alkoxy-6-acyloxymethyl 2H-pyran-3(6H)-one is an appropriate alkyl group or an aryl group such as methyl, ethyl, n-propyl, isopropyl, tert-butyl, phenyl, and benzyl group. The R¹ is removed from the system by the oxidation reaction, and is preferably isopropyl, phenyl, or tert-butyl group, but not restricted thereto. R² is a protection group for primary hydroxyl groups, and is preferably an acyl group such as acetyl, propionyl, benzoyl, p-chlorobenzoyl group, but not restricted thereto. With a consideration that the subject compound (II) is an important intermediate for various kinds of useful compounds and may be converted into the useful compounds, R² is also preferably a protection group such as tert-butyldimethyl silyl group and p-toluensulphonyl group accordingly.

According to the invention, the starting material (I) is known and can be produced from D-glucose through a combination of known reactions. In a first method, a combination of a reaction disclosed in Nature, 165,369 (1950) and that in J.Org.Chem., 54,2103 is utilized. An example of synthesis according to the first method is described in detail in a reference example 1 later. A synthetic route in the reference example 1 is described in the following a flow chart 1:

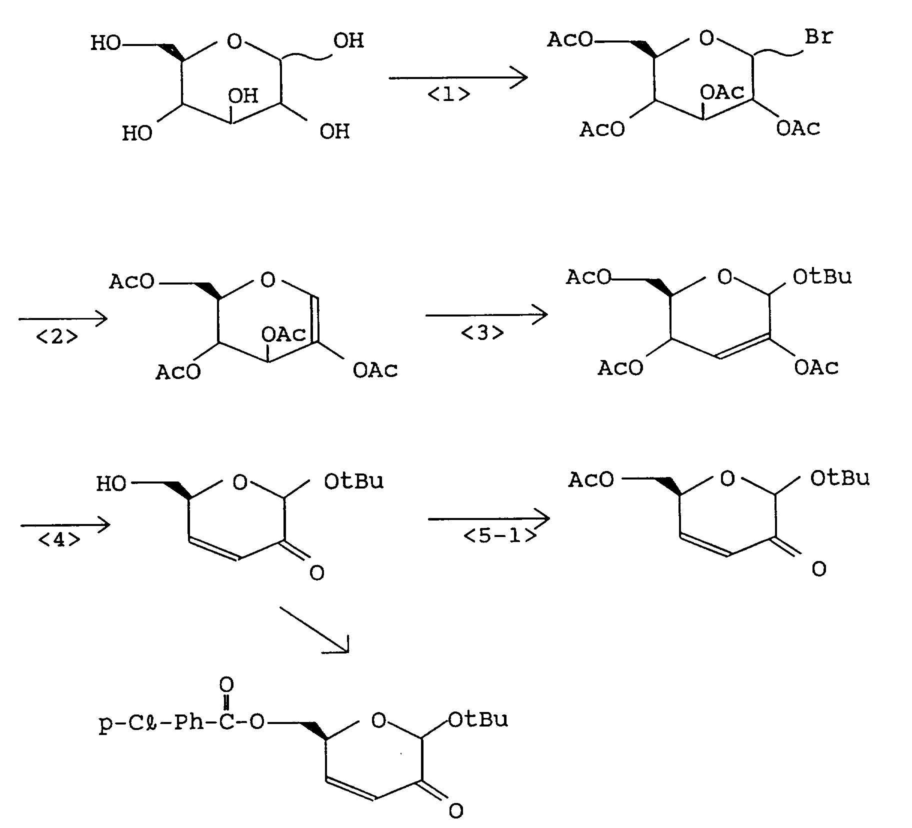

According to a second method, a combination of a reaction disclosed in Nature, 165,369 (1950), a reaction disclosed in Carbohydrate Research, 167,189 (1987), and that in Tetrahedron, 46,231 (1990) is utilized. An examples of synthesis according to the second method is described in detail in a reference example 2 later. A synthetic route in the reference example 2 is shown in the following flow chart 2:

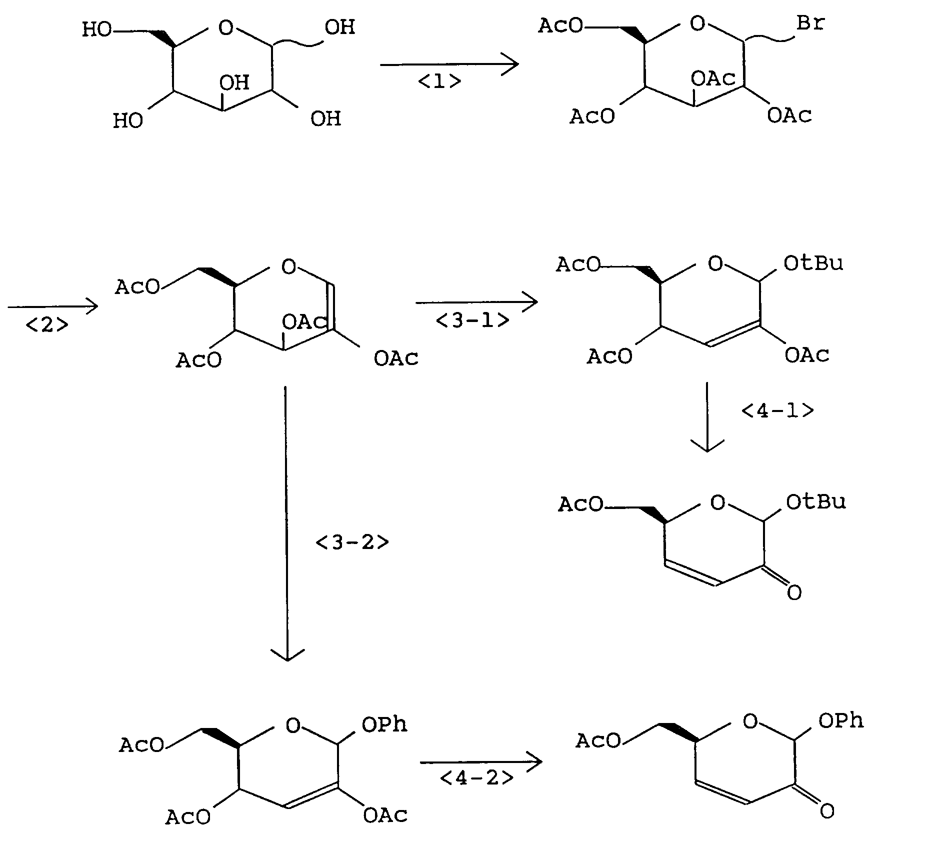

According to the invention, peracid for oxidation of the starting material (I) is not restricted. However, examples of the peracid preferably include peracetic acid, performic acid, perbenzoic acid, perphthalic acid, metachloro perbenzoate, and magnesium monoperoxyphthalate hexahydrate, more preferably, peracetic acid and magnesium monoperoxyphthalate hexahydrate, and most preferably, peracetic acid.

According to the invention, equimolecular quantities of the starting material (I) and peracetic acid are reacted. One mole of peracetic acid is therefore stoichiometry sufficient for one mole of the starting material. However, 1.0 - 3.0 moles, preferably, 1.2 - 1.5 moles of peracetic acid is utilized to react with one mole of the starting material.

According to the invention, the oxidation reaction is made in a liquid phase. Examples of a solvent in the oxidation reaction include a commonly used organic solvent such as acetic acid, methylene chloride, and chloroform. When a particular solvent is selected, such a solvent should meet the following requirements; being capable of readily dissolving 2-alkoxy-6-acyloxymethyl 2H-pyran-3(6H)-one, not reacting with peracid, and not making post-treatment after reaction difficult. Any solvent may be used, as far as it satisfies these conditions.

The oxidation reaction as stated above can be easily performed, namely, by adding peracid to the starting material (I) and stirring the resultant mixture at room temperature, preferably at 15 to 25°C for 10 to 18 hours for reaction.

The subject compound (II) thus obtained can be straightly used as an intermediate for synthesis of pharmaceutical drugs as stated above. The compound (II) can be further purified in the following manner in order to obtain a subject compound (II) of higher purity; adding sodium sulfite solution to the reaction mixture in order to reduce an excessive amount of peracids, adding sodium bicarbonate for neutralization, extracting with an organic solvent such as dichloromethane and chloroform, drying the organic phase with a drying agent such as sodium sulphate, and evaporating the solvent under reduced pressure.

As sated above, since the starting material (I) of the invention can be prepared from D-glucose which is abundant in nature, (S)-γ-acyloxymethyl-α,β-butenolide of the invention can be produced at high yield. Moreover, the invention utilizes commonly used organic synthesis throughout all steps. The subject compound can be thus easily produced in an industrial scale.

This invention therefore eminently contributes to a field for production of useful compounds such as prostaglandin, antileukemic lignan, and antibiotic rasaroside A, in which field the subject compound is utilized as a starting material.

### [Best Mode of Carrying out the Invention]

Detailed description of the invention is as follows by way of examples.

### Example 1

In this example, such a starting material (I) wherein R¹ is tert-butoxy group and R² is acyl group, i.e., 2-tert-butoxy-6-acetoxymethyl 2H-pyran-3(6H)-one was used.

To 300 ml of acetic acid, 35g of the starting material (I) as said was added and dissolved therein. To the resultant solution, 26g of 50% peracetic acid (acetic acid solution) was added, and the obtained solution was stirred at room temperature for 15 hours. After the reaction was terminated, 100 ml of saturated sodium sulfite solution was added to the reacted solution in order to reduce an excessive amount of peracids. Next, saturated sodium bicarbonate was slowly added to the reacted solution for neutralization. Next, 500 ml of chloroform was added to the neutralized solution for extraction. The obtained extraction layer was dried with sodium sulphate, and evaporated under reduced pressure in order to remove the solvent. Thus, 18g of (S)-γ-acetoxymethyl-α,β-butenolide was obtained. Yield was 80%.

Melting point, specific rotation, and IR and ¹H-NMR data of (S)-γ-acetoxymethyl-α,β-butenolide was found to coincide with those described in Heterocycles, 31,423 (199O).

### Example 2

In this example, such a starting material (I) wherein R¹ is tert-butoxy group and R² is p-chlorobenzoyl group, i.e., 2-tert-butoxy-6-p-chlorobenzoyloxymethyl 2H-pyran-3(6H)-one was utilized.

To 50 ml of acetic acid, 4.9g of the aforementioned starting material (I) was dissolved. To the resultant solution, 2.6g of 50% peracetic acid (acetic acid solution) was added, and the obtained solution was stirred at room temperature for 15 hours for reaction. After the reaction was terminated, 50 ml of saturated sodium sulfite solution was added to the reacted solution in order to reduce an excessive amount of peroxides. Next, to the solution, a saturated sodium bicarbonate solution was slowly added for neutralization, and 100 ml of chloroform was further added for extraction. The obtained extraction layer was dried with sodium sulphate, and evaporated under reduced pressure in order to remove the solvent. Thus, 3g of (S)-γ-p-chlorobenzoyloxymethyl-α,β-butenolide was obtained. Yield was 82%.

Structure of (S)-γ-p-chlorobenzoyloxymethyl-α,β-butenolide was determined by means of ¹H-NMR.
¹H - NMR (ppm from TMS)
4.61 (2H, m),
5.40 (1H, m),
6.23 (1H, dd, J = 2.1, 5.8 Hz),
7.44 (2H, d, J = 8.6 Hz),
7.60 (1H, m),
7.98 (1H, d, J = 8.6 Hz)

### Example 3

In this example, such a starting material (I) wherein R¹ is phenyl group and R² is acetyl group, i.e., 2-phenyl-6-acetoxymethyl 2H-pyran-3(6H)-one was utilized.

To 50 ml of acetic acid, 5.2g of the aforementioned starting material (I) was added and dissolved therein. To the resultant solution, 2.6g of 50% peracetic acid (acetic acid solution) was added, and the obtained solution was stirred at room temperature for 15 hours for reaction. After the reaction was terminated, 50 ml of saturated sodium sulfite solution was added to the solution in order to reduce an excessive amount of peroxides. Next, saturated sodium bicarbonate was slowly added to the reacted solution for neutralization, and 100 ml of chloroform was further added for extraction. The obtained extraction layer was dried with sodium sulphate, and evaporated under reduced pressure in order to remove the solvent. Thus, 1.7g of (S)-γ-acetoxymethyl-α,β-butenolide was obtained. Yield was 55%.

Melting point, specific rotation, and IR and ¹H-NMR data of (S)-γ-acetoxymethyl-α,β-butenolide thus obtained were found to coincide with those described in Heterocycles, 31,423 (199O).

Structure of the starting material (I), i.e., 2-phenyl-6-acetoxymethyl 2H-pyran-3(6H)-one was determined by means of IR and ¹H-NMR.
IR (cm⁻¹)
1744, 1702, 1593
¹H-NMR (ppm from TMS)
2.08 (3H, s),
4.26 (1H, dd, J = 4.5, 11.7 Hz),
4.40 (1H, dd, J = 5.2, 11.7 Hz),
4.48 (1H, m),
5.58 (1H, s),
6.31 (1H, dd, J = 2.1, 10.7 Hz),
7.0 - 7.4 (6H, m),

Next, examples of synthesis of the starting material (I) from D-glucose are described as reference examples as follows.

### Reference Example 1:

### Example for Production of 2-tert-butoxy-6-acetoxymethyl 2H-pyran-3(6H)-one from anhydrous D-glucose, No. 1

### <Step 1>

step for production of tetra-O-acetyl-α-D-glucopyranosyl bromide from anhydrous D-glucose; Nature, 165,369 (1950)

To a 1ℓ-three necked flask, 400 ml of anhydrous acetic acid was introduced, followed by cooling on ice. To this flask, 2.4 ml of 65% perchloric acid was dropwise added. The temperature of the resultant solution was returned to room temperature, and 100g of anhydrous D-glucose was added to the solution while stirring in order to maintain the temperature of the solution at 30 to 40°C. Then, the reacted solution was cooled to 20°C, and added with 30g of red phosphorus. Next, to the resultant solution, 58 ml of bromine was added dropwise with the temperature of the solution at 20°C or less maintained. Subsequently, 36 ml of water was dropwise added to the obtained solution. The reacted solution was then allowed to stand at room temperature for two hours, added with chloroform and filtered to remove the resultant precipitants. To the filtrate, cooled water was added. The mixture was separated into organic and inorganic layer, and chloroform layer was separately obtained. This layer was washed with water and saturated sodium bicarbonate solution in this order, dried with anhydrous magnesium sulphate, and evaporated under reduced pressure to obtain a solid crystal. This crystal was recrystallized from ether to afford 190g of tetra-O-acetyl-α-D-glucopyranosyl bromide. Yield was 83%.

### <Step 2>

step for production of 2,3,4,6-tetra-0-acetyl-1,5-anhydro-D-arabinohex-1-enitol from tetra-O-acetyl-α-D-glucopyranosyl bromide; J.Org.Chem., 54,2103 (1989)

To a 1ℓ-three necked flask, 190g of tetra-O-acetyl-α-D-glucopyranosyl bromide obtained in the step 1, 19g of tetrabutylammonium bromide, and 250 ml of dried acetonitrile were introduced. To this flask, 145 ml of diethylamine was slowly added dropwise over two hours. After the addition was terminated, the resultant solution was stirred at 0°C for 15 minutes, and additionally stirred at room temperature for one hour. To the obtained black solution, 250 ml of chloroform was added. The obtained chloroform layer was washed with 320 ml of saturated sodium bicarbonate, dried with sodium sulphate, and evaporated under reduced pressure to remove the solvent. The resultant residue was subjected to a silica gel column chromatography (hexan:ethyl acetate = 7:3). The obtained substance was recrystallized from a mixture solvent of ether/hexan, and 68g of 2,3,4,6-tetra-O-acetyl-1,5-anhydro-D-arabinohex-1-enitol was thus obtained. Yield was 42%.

### <Step 3>

step for production of tert-butyl 2,4,6-tri-O-acetyl-3-deoxy-α-D-erythrohex-2-enopyranoside from 2,3,4,6-tetra-O-acetyl-1,5-anhydro-D-arabinohex-1-enitol; J.Org.Chem., 54,2103 (1989)

In a 500ml-three necked flask, 68g of 2,3,4,6-tetra-O-acetyl-1,5-anhydro-D-arabinohex-1-enitol was dissolved in 140 ml of dried dichloromethane containing 80 ml of tert-butanol. The resultant solution was cooled to 0°C, and dropwise added with 13 ml of trifluoroboran/ether complex. The obtained solution was stirred at room temperature for 40 hours for reaction, and the reacted solution was diluted with 350 ml of dichloromethane. Subsequently, 180 ml of saturated sodium bicarbonate solution was slowly added to the diluted solution, followed by stirring for five minutes. The obtained dichloromethane layer was separated, washed with saturated saline, and evaporated under reduced pressure to remove the solvent. The obtained residue was subjected to a silica gel column chromatography (hexan:ethyl acetate = 8:2). The obtained substance was then recrystallized from a ethanol solvent. Thus, 62g of tert-butyl 2,4,6-tri-O-acetyl-3-deoxy-α-erythrohex-2-enopyranoside was obtained. Yield was 87%.

### <Step 4>

step for production of 2-tert-butoxy-6-hydroxymethyl 2H-pyran-3(6H)-one from tert-butyl 2,4,6-tri-O-acetyl-3-deoxy-α-D-erythrohex-2-enopyranoside; J.Org.Chem., 54,2103 (1989)

To 120ml of methanol, 62g of tert-butyl 2,4,6-tri-O-acetyl-3-deoxy-α-D-erythrohex-2-enopyranoside prepared in the step 3 was added for dissolution. The obtained solution was added to 3ℓ of a mixture solution containing ethyleneglycol, triethylamine, and water (mixture ratio = 1:1:3). The obtained mixture was stirred at room temperature for reaction, and added with 1ℓ of methylene chloride for extraction. The resultant organic layer was washed with saturated saline, dried with sodium sulphate, and evaporated under reduced pressure in order to remove the solvent. The obtained residue was subjected to a silica gel column (hexane:ethyl acetate = 6:4). Thus, 14g of 2-tert-butoxy-6-hydroxymethyl 2H-pyran-3(6H)-one was obtained. Yield was 40%.

### <Step 5-1>

step for production of 2-tert-butoxy-6-acetoxymethyl 2H-pyran-3(6H)-one from 2-tert-butoxy-6-hydroxymethyl 2H-pyran-3(6H)-one;

To a mixture solution containing 500 ml of pyridine and 300 ml of anhydrous acetic acid, 14g of 2-tert-butoxy-6-hydroxymethyl 2H-pyran-3(6H)-one prepared in the step 4 was added for dissolution. The resultant solution was stirred at room temperature for five hours for reaction. Then, to the reacted solution, 1ℓ of methylene chloride and 1ℓ of water were added for extraction. The obtained organic layer was washed with saturated saline, dried with sodium sulphate, and evaporated under reduced pressure to remove the solvent. Thus, 14g of 2-tert-butoxy-6-acetoxymethyl 2H-pyran-3(6H)-one was obtained. Yield was 82%. ¹H-NMR data for this product was obtained as follows;
¹H - NMR ; (ppm form TMS)
1.31 (9H, s), 2.09 (3H, s),
4.22 (1H, dd, J = 4.5, 11.6 Hz),
4.35 (1H, dd, J = 5.8, 11.6 Hz),
4.85 (1H, m), 5.13 (1H, s),
6.15 (1H, dd, J = 2.5, 10,6 Hz),
6.98 (1H, dd, J = 1.6, 10.6 Hz)

### <Step 5-2>

step for production of 2-tert-butoxy-6-p-chlorobenzoyloxymethyl 2H-pyran-3(6H)-one from 2-tert-butoxy-6-hydroxymethyl 2H-pyran-3(6H)-one;

To 200 ml of dried dichloromethane, 10g of 2-tert-butoxy-6-hydroxymethyl 2H-pyran-3(6H)-one obtained in the step 4 was added for dissolution. To the resultant solution, 10g of p-chlorobenzoyl chloride, 5.8g of triethylamine, and 1.2g of 4-N,N-dimethylaminopyridine were added, followed by stirring at room temperature for five hours for reaction. To the reacted solution, 500 ml of methylene chloride and 500 ml of water were added for extraction. The obtained organic layer was washed with saturated saline, dried with sodium sulphate, and evaporated under reduced pressure to remove the solvent. Thus, 14g of 2-tert-butoxy-6-p-chlorobenzoyloxymethyl 2H-pyran-3(6H)-one was obtained. Yield was 82%. ¹H-NMR data of this product was as follows;
¹H - NMR ; (ppm from TMS)
1.31 (9H, s),
4.49 (1H, dd, J = 5.7, 11.6 Hz),
4.56 (1H, dd, J = 4.5, 11.6 Hz),
5.00 (1H, m), 5.17 (1H, s),
6.20 (1H, dd, J = 2.6, 10.6 Hz),
7.05 (1H, dd, J = 1.9, 106 Hz),
7.41 (1H, d, J = 8.6 Hz),
7.96 (1H, d, J = 8.6 Hz)

### Reference Example 2:

### Example of Production of 2-tert-butoxy-6-acetoxymethyl 2H-pyran-3(6H)-one from anhydrous D-glucose, No. 2

### <Step 1>

step for production of tetra-O-acetyl-α-D-glucopyranosyl bromide from anhydrous D-glucose; Nature, 165,369 (1950)

According to the same manner as that in the step 1 of the reference Example 1, tetra-O-acetyl-α-D-glucopyranosyl bromide was prepared from anhydrous D-glucose. Yield was 85%.

### <Step 2>

step for production of 2,3,4,6-tetra-O-acetyl-1,5-anhydro-D-arabinohex-1-enitol from tetra-O-acetyl-α-D-glucopyranosyl bromide; Carbohydrate Research, 167,187 (1989)

To 5 ml of 1,2-dichloroethane, 3.0g (8.5 mmol) of tetra-O-acetyl-α-D-glucopyranosyl bromide obtained in the step 1 was added for dissolution. The resultant solution was cooled to -20°C. To this solution, 1.45g (9.5 mmol) of 1,8-diazabicyclo(5,4,0)-7-undecene(DBU) was added dropwise, followed by stirring for thirty minutes. The reaction solution was returned to room temperature, and further stirred for one hour. Next, to the reacted solution, 100 ml of dichloromethane was added, followed by addition of 5% chloric acid. The obtained organic layer was separated, washed with saturated sodium bicarbonate, dried with sodium sulphate, and evaporated under reduced pressure to remove the solvent. The obtained residue was recrystallized from ethanol. Thus, 1.95g of 2,3,4,6-tetra-O-acetyl-1,5-anhydro-D-arabinohex-1-enitol was obtained. Yield was 83%.

### <Step 3-1>

step for production of tert-butyl 2,4,6-tri-O-acetyl-3-deoxy-α-D-erythrohex-2-enopyranoside from 2,3,4,6-tetra-O-acetyl-1,5-anhydro-D-arabinohex-1-enitol; J.Org.Chem., 54,2103 (1989)

According to the same manner as that in the step 3 in the reference Example 1, tert-butyl 2,4,6-tri-O-acetyl-3-deoxy-α-erythrohexy-2-enopyranoside was obtained from 2,3,4,6-tetra-O-acetyl-1,5-anhydro-D-arabinohex-1-enitol. Yield was 87%.

### <Step 4-1>

step for production of 2-tert-butoxy-6-acetoxymethyl 2H-pyran-3(6H)-one from tert-butyl 2,4,6-tri-O-acetyl-3-deoxy-α-D-erythrohex-2-enopyranoside; Tetrahedron, 46,231 (1990)

To 120 ml of ethanol, 10.2g (29.7 mmol) of tert-butyl 2,4,6-tri-O-acetyl-3-deoxyα-D-erythrohex-2-enopyranoside obtained in the step 3 was added for dissolution. The resultant solution was cooled to -20°C. To the cooled solution, 4.09g of potassium carbonate was added, followed by stirring for one hour. After the reaction was terminated, cationic ion-exchange resin (Dowex-50) was added to the solution in order to remove potassium ions. The solution was then evaporated under reduced pressure to remove the solvent. The obtained oily substance was dissolved in ether. The obtained solution was washed with saturated sodium bicarbonate solution, dried with sodium sulphate, and evaporated under reduced pressure to remove the solvent. An oily substance was thereby obtained.

This oily substance contained, in addition to the objective substance which is 2-tert-butoxy-6-acetoxymethyl 2H-pyran-3(6H)-one, derivatives of the compounds whose acetyl groups are partially hydrolyzed. These hydrolyzed compounds were then conversed into the objective compound according to the following procedure in order to increase yield.

First, the oily substance as obtained was dissolved in 20 ml of dichloromethane. To the resultant solution, 2 ml of anhydrous acetic acid, 0.1g of N,N-dimethylaminopyridine, and 2 ml of pyridine were added, followed by stirring for thirty minutes. After the reaction was terminated, water was added and the organic phases was separated. The obtained organic layer was washed with saturated sodium bicarbonate, dried with sodium sulphate, and evaporated under reduced pressure to remove the solvent. The obtained residue was subjected to a silica gel column chromatography (hexane:acetic acid = 9:1). Thus, 5.0g of the objective compound was obtained. Yield was 70%.

### <Step 3-2>

step for production of phenyl 2,4,6-tri-O-acetyl-3-deoxy-α-D-erythrohex-2-enopyranoside from 2,3,4,6-tetra-O-acetyl-1,5-anhydro-D-arabinohex-1-enitol;

According to the same manner as that in the step 3 in the reference Example 1, 2,3,4,6-tetra-O-acetyl-1,5-anhydro-D-arabinohex-1-enitol and phenol were made to react, with the exception that tert-butanol was replaced with phenol and the reaction temperature and the reaction time were made 0°C and one hour, respectively. Phenyl 2,4,6-tri-O-acetyl-3-deoxy-α-erythrohex-2-enopyranoside was thus obtained as needle crystal. Melting point of this substance was 47 to 48 °C. Yield was 95%.

Structure of this product was determined by means of IR and ¹H-NMR.
IR (cm⁻¹)
1740
¹H - NMR (ppm from TMS)
1.99 (3H, s),
2.11 (3H, s),
2.18 (3H, s),
4.1 - 4.4 (3H, m)
5.54 (1H, dd, J = 2.1, 9.3 Hz),
5.69 (1H, s),
5.99 (1H, d, J = 2.1 Hz),
7.0 - 7.3 (5H, m)

### <Step 4-2>

step for production of 2-phenyl-6-acetoxymethyl 2H-pyran-3(6H)-one from phenyl 2,4,6-tri-O-acetyl-3-deoxyα-D-erythrohex-2-enopyranoside

According to the same manner as that in the step 4-1, 2-phenyl-6-acetoxymethyl 2H-pyran-3(6H)-one was obtained from phenyl 2,4,6-tri-O-acetyl-3-deoxy-α-D-erythrohex-2-enopyranoside. Yield was 67%.

## Claims

1. A method for producing (S)-γ-acyloxymethyl-α,β-butenolide comprising a step in which a starting material selected from the group consisting of (6S)-2-alkoxy-6-acyloxymethyl-2H-pyran-3(6H)-one and (6S)-2-aryloxy-6-acyloxymethyl-2H-pyran-3(6H)-one is oxidized with peracid in an organic solvent.

2. A method according to claim 1, characterized in that said peracid is selected from a group consisting of peracetic acid, metachloro perbenzoic acid, and magnesium monoperoxyphthalate hexahydrate.

3. A method according to claim 1, characterized in that an amount of said peracid is 1.0 to 3.0 moles per one mole of said starting material.

4. A method according to claim 1, characterized in that said organic solvent can readily dissolve the starting material, does not react with said peracid, and does not produce byproducts which make treatment after reaction difficult.

5. A method according to claim 4, characterized in that said organic solvent is selected from a group consisting of acetic acid, methylene chloride, and chloroform.

## Patentansprüche

1. Verfahren zur Herstellung von (S)-γ-Acyloxymethyl-α,β-butenolid mit einem Schritt, bei dem ein Ausgangsmterial, ausgewählt aus der Gruppe, bestehend aus (6S)-2-Alkoxy-6-acyloxymethyl-2H-pyran-3(6H)-on und (6S)-2-Aryloxy-6-acyloxymethyl-2H-pyran-3(6H)-on mit einer Persäure in einem organischen Lösungsmittel oxidiert wird.

2. Verfahren nach Anspruch 1,
**dadurch gekennzeichnet,**
daß die Persäure aus einer Gruppe, bestehend aus Peressigsäure, Metachlorperbenzoesäure und Magnesiummonoperoxyphthalathexahydrat, ausgewählt wird.

3. Verfahren nach Anspruch 1,
**dadurch gekennzeichnet,**
daß die Menge der Persäure 1,0 bis 3,0 Mole pro einem Mol des Ausgangsmaterials beträgt.

4. Verfahren nach Anspruch 1,
**dadurch gekennzeichnet,**
daß das organische Lösungsmittel das Ausgangsmaterial leicht löst, mit der Persäure nicht reagiert und keine Nebenprodukte ergibt, die die Behandlung nach der Reaktion schwierig gestalten.

5. Verfahren nach Anspruch 4,
**dadurch gekennzeichnet,**
daß das organische Lösungsmittel aus einer Gruppe, bestehend aus Essigsäure, Methylenchlorid und Chloroform, ausgewählt wird.

## Revendications

1. Méthode de production de (S)-γ-acyloxyméthyl-α,β-buténolides comprenant une étape dans laquelle une matière première choisie dans le groupe constitué par les (6S)-2-alcoxy-6-acyloxyméthyl-2H-pyrann-3(6H)-ones et les (6S)-2-aryloxy-6-acyloxyméthyl-2H-pyrann-3(6H)-ones est oxydée par un peracide dans un solvant organique.

2. Méthode selon la revendication 1, caractérisée en ce que ledit peracide est choisi dans le groupe constitué par l'acide peracétique, l'acide métachloroperbenzoïque, et le monoperoxyphtalate de magnésium hexahydraté.

3. Méthode selon la revendication 1, caractérisée en ce que la quantité dudit peracide va de 1,0 à 3,0 moles pour une mole de ladite matière première.

4. Méthode selon la revendication 1, caractérisée en ce que ledit solvant organique peut dissoudre facilement la matière première, ne réagit pas avec ledit peracide et ne produit pas de sous-produits rendant le traitement après réaction difficile.

5. Méthode selon la revendication 4, caractérisée en ce que ledit solvant organique est choisi dans le groupe constitué par l'acide acétique, le chlorure de méthylène et le chloroforme.
